# EUROPEAN PATENT APPLICATION

(11) **EP 4 202 425 A1**
(43) Date of publication of application: **28.06.2023**
(21) Application number: 21217226.6
(22) Date of filing: 23.12.2021
(51) Int. Cl.: G01N 23/046, G01N 33/483

(54) **PHANTOM FOR TESTING DRUG-CELL INTERACTION**

(71) Applicant: King Faisal Specialist Hospital & Research Centre, Riyadh, 11211 (SA)
(72) Inventor: AlQahtani, Mohammed, 11211 Riadh (SA); AlMalki, Yousif, 11211 Riadh (SA); Schneider, John, 11211 Riadh (SA)
(74) Representative: Hartig, Michael

(57) **Abstract**

The present invention provides a device, system, use, and method for testing accumulation of radiotracer molecules to biological cells, and more particularly, for testing cell uptake and saturation. Therein, the device comprises a receptacle having an inlet, an outlet, and an opening for receiving a specimen carrier. The device further comprises the specimen carrier that comprises means for holding a specimen and is configured such that after loading of the specimen carrier with a specimen and insertion of the specimen carrier into the receptacle, the specimen being in fluid connection with the interior of the receptacle.

## Description

The present invention generally relates to a phantom for testing drug-cell interaction, and more particularly, to a device, system, and method for testing accumulation of radiotracer molecules to biological cells. The present invention further relates to the use of a device in Positron Emission Tomography (PET) or Single Photon Emission Computed Tomography (SPECT) for testing accumulation of radiotracer molecules to biological cells, and more particularly, cell uptake and saturation.

In current practice, Positron Emission Tomography (PET) and Single Photon Emission Computed Tomography (SPECT) cameras are employed as non-invasive imaging tools to investigate disease progression, assess treatment efficacy, and develop new radiotracers and pharmaceuticals or drugs for clinical use. More particularly, PET and SPECT are commonly used in oncology for diagnosing tumors, staging, and monitoring radiotherapy. In cardiology, PET and SPECT are employed for investigating myocardial viability and blood perfusion. In neurology, the imaging techniques are commonly used to assess blood perfusion and oxygen consumption. On that account, PET and SPECT pertain to be strong molecular imaging modalities due to the availability and ongoing development of radionuclides that have suitable physical, physiological, and biochemical characteristics. In general, performance testing is required before PET and SPECT systems can be put in use. So-called phantoms are conventional tools for such quality control/assurance testing. Hence, imaging a phantom is usually performed before imaging the actual subject of interest to appropriately adjust the camera settings. In the state of the art, the test subjects for investigating disease mechanism and pre-clinical drug testing with PET or SPECT cameras are small-animal models injected with so-called radiotracers or radiotracer molecules, i.e. chemical compounds or biomolecules comprising a radionuclide. However, the current devices, systems, uses, and methods have certain shortcomings such as the difficulty to predict the drug response in the small-animal model to new radiotracer molecules and hence, initial ill-definition of the required amount of radiotracer molecules for injection. As a consequence, ethical considerations arise that complicate and inhibit larger-scale investigations.

On the other hand, cell cultures have gained increasing interest for the investigation of disease mechanisms and pre-clinical drug testing. In particular, the use of cell cultures allows studying cell function and dysfunction, and identifying targets for drug screening. Among other applications, cell cultures can be employed for testing drug metabolism and cytotoxicity, antibody production, vaccine production, cancer pharmacogenomics to predict clinical response, developing new pharmacogenomic hypotheses for further testing, and identifying novel mechanisms associated with variations in drug response. However, the devices, systems, uses, and methods of the state of the art employed for studies on cell cultures have certain shortcomings such as that the required lab procedures are lengthy, labor intense, and involve a high level of personal intervention by the experimenting personnel. This sets down the reliability and reproducibility of the testing results and likewise, complicates and inhibits larger-scale investigations.

It is therefore the technical problem underlying the present invention to provide a device, system, use, and method with automation and high-throughput capabilities for efficient and reliable testing of drug-cell interaction that overcomes the shortcomings of the devices, systems, uses, and methods of prior art for testing cell uptake, saturation, binding, blocking, or activating.

The technical problem is solved by a device, system, use, and method as defined in claims 1, 8, 12, and 13, respectively. Advantageous embodiments of the invention are defined in the dependent claims.

In general, the present invention provides a device for testing the accumulation of radiotracer molecules on biological cells. Therein, the device comprises a receptacle having an inlet, an outlet, and an opening for receiving a specimen carrier. The device further comprises the specimen carrier that comprises means for holding a specimen and is configured such that after loading of the specimen carrier with a specimen and insertion of the specimen carrier into the receptacle, the specimen being in fluid connection with the interior of the receptacle. Therein, the specimen carrier or the specimen comprises a filter means such as a filter paper, fabric, or membrane including gel membranes.

In an embodiment, the specimen is in fluid connection with the interior of the receptacle through the filter means.

In yet another embodiment, the device comprises a receptacle with two or more openings for receiving two or more specimen carriers, respectively.

According to an embodiment, the specimen comprises cells absorbed on a filter means such as a filter paper, fabric, or membrane including gel membranes. The specimen may also be or comprise a tissue section. More particularly, the tissue section may not comprise a filter paper. The specimen carrier may be loaded with the cells or tissue section shortly before use. It is conceivable that the cells can be loaded and absorbed on the filter means externally, for example, by using a syringe body and an HPLC solvent line filter head. The filter paper with the absorbed cells is then accommodated by the specimen carrier. Alternatively, the cells may be loaded directly onto the filter means already held in the specimen carrier. In this case, the inlet may be closed and the interior of the specimen carrier may be closed on any other surface than a surface comprising the filter means. Access of cell media are then removed by applying a slow vacuum pulse through the outlet of the receptacle for a short duration, i.e. seconds.

Tissue sections may be patient-derived or derived from an animal model. It is conceivable that the specimen carrier may comprise a basket, clamp, mesh, patch, filter means, or any type of container or support suitable for holding a specimen such as one described above. As an example, the filter means is a filter paper for holding the cells and may be a glass fiber filter paper such as a WHATMAN filter paper, which offers the high quality and reproducibility, and are recognized as the industry standard for receptor binding, lymphocyte studies, and other harvesting applications. WHATMAN filter papers are produced from 100% borosilicate glass without the use of any adhesive or binder.

A crucial part of drug-cell interaction and a prerequisite for an appropriate drug response is the accumulation of the target drug to the biological cells in a specimen, which is assessed in cell uptake, saturation, binding, blocking, or activating studies. More particularly, the target drug has to be delivered to the cells and undergo binding or cell uptake to saturate the cells. The above-defined device according to the present invention allows directly observing the accumulation of a target drug, i.e. radiotracer molecules, to biological cells in a specimen. More particularly, the device according to the present invention allows testing cell uptake and saturation. Since the specimen is in fluid connection with the interior of the receptacle, radiotracer molecules filled into the interior of the receptacle can be delivered to the cells in the specimen and undergo binding or cell uptake. The device is suitable for investigating the accumulation mechanisms by detecting the radiation coming from the biological cells in the specimen. The radiation may be particle radiation or electromagnetic radiation coming from the cells in the specimen, and more particularly, coming from the emitting radiotracer molecules the cells are saturated with. Depending on the radiotracer molecules, the emitted radiation may be electromagnetic radiation such as visible light, ultraviolet, x-ray or gamma radiation or particle radiation such as beta particles or alpha particles. In case the radiotracer molecules comprise a radionuclide or radioactive isotope, the measuring of the level of radioactivity in the cells can then lead to insights into the underlying accumulation mechanisms. No additional measures - such as complicated manual optical-investigation, washing or centrifugation/re-suspension procedures - are needed before, during, or after data acquisition that would require intervention of experimenting personnel and that would put the cells under additional stress. Hence, the device according to the present invention provides automation and high-throughput capabilities for efficient and reliable testing of drug-cell interaction.

According to an embodiment of the invention, the device further comprises a pump means configured for a connection with the inlet to direct a fluid through the receptacle and past the specimen.

Hence, the device with the above-described features provides capabilities to mimic blood flow and allows for diffusive and convective delivery of the radiotracer molecules and nutrients to the specimen. Consequently, even though the accumulation of radiotracer molecules to the cells present in the specimen is tested in-vitro, i.e. outside the influence of the normal physiological conditions present in the body, a more natural environment can be provided for the cells and the informative value of the testing results can be improved. Further, it is conceivable that the pump means can be used to flush the receptacle with a fluid without comprised radiotracer molecules, for example, before starting the testing or after a predetermined waiting time after filling the radiotracer molecules into the receptacle. Hence, the device allows detecting radiation coming from the saturated cells in the specimen in a state where the receptacle is filled with a fluid that does not contain any radiotracer molecules. Suitable pump means, for example, include microfluidic pressure pumps, manually operated syringes such as shielded syringes, or programmable syringe pumps. It is thinkable that the inlet and outlet have the form of a hole or hose barb connector or comprise means for a screw connection for plugging a tubing or syringe.

In another embodiment, the specimen carrier comprises an inner space that can be closed by the filter means, and is configured such that after loading of the specimen into the inner space of the specimen carrier and insertion of the specimen carrier into the receptacle, the specimen is in fluid connection with the interior of the receptacle through the filter means. In particular, the fluid connection between the interior of the receptacle and the specimen may be realized exclusively through the filter means.

It is thinkable that the inner space of the specimen carrier will hold the filter means or the filter paper with the absorbed cells shortly before use. Further, the interior of the receptacle may have a volume in the range of 0.1 ml to 100 ml. The filter paper may have a circular or circular symmetrical shape with a diameter in the range of 5 mm to 50 mm depending on the dimensions of the specimen carrier. It is conceivable that the filter means comprises a membranous material with pore size of ≤ 1.0 µm. For example, WHATMAN filter papers may be used. WHATMAN filter papers are glass fibre filter papers that offer high quality and reproducibility, and are recognized as the industry standard for receptor binding, lymphocyte studies, and other harvesting applications. Whatman papers are produced from 100% borosilicate glass without the use of any adhesive or binder). Further, the material of the filter means may be hydrophobic or hydrophilic, and may have certain permeability and sorption characteristics for specific chemical compounds and biomolecules. Hence, the delivery of the radiotracer molecules to the specimen, as well as nutrient supply, can be controlled by choosing an appropriate filter-means material, i.e. choosing the filter-means material in terms of pore size, sorption, permeability, hydrophobicity or hydrophilicity characteristics. Moreover, after the specimen carrier is loaded with the specimen, fungal or bacterial contamination of the specimen can be prevented. Further, it is conceivable that the specimen can be inserted with the filter means and discarded with the filter means before and after use, respectively, which increases throughput and reduces safety risks for the experimenting personnel.

In a further embodiment, the specimen carrier comprises the shape of a tube with an opening at at least one end thereof, wherein a filter means can be connected with the specimen carrier so that the opening at the at least one end is sealed.

In yet another embodiment, the filter means has a pore size such that the filter means is substantially permeable for the radiotracer molecules and substantially non-permeable for the biological cells, wherein the radiotracer molecules comprise a chemical compound or a biomolecule with a radionuclide, and the specimen comprises the biological cells.

Since there is a difference in permeability of the filter means for the radiotracer molecules and the biological cells, the device enables the selective delivery of the radiotracer molecules to the specimen and at the same time, retains the biological cells in the specimen carrier. It is conceivable that the radiotracer molecules comprise a chemical compound or biomolecule relevant for a biological function or receptors on the cells. Examples for suitable chemical compounds and biomolecules are monosaccharides, in particular glucose and glucose analogues, water, ammonia, nucleotides, nucleosides, nucleic acids, amino acids, polypeptides, and proteins.

According to an embodiment, the radiotracer molecules each comprise a radionuclide. A radionuclide, i.e. radioactive isotope of an element, is incorporated into the chemical compound or biomolecule and emits a positron or gamma ray upon radioactive decay.

Examples for radionuclides suitable for PET are ¹¹C, ¹³N, ¹⁵O, ¹⁸F, ⁴⁴Sc, ⁴⁵Ti, ⁵²Mn, ⁵⁵Co, ⁶⁰Cu, ⁶²Cu, ⁶⁴Cu, ⁶⁸Ga, ⁷⁶Br, ⁸²Rb, ⁸⁶Y, ⁸⁹Zr, ^{94m}Tc, ¹²²I, and ¹²⁴I. Examples for radionuclides suitable for SPECT are ⁵¹Cr, 57Co, ⁶⁷Ga, ⁷⁵Se, ^{81m}Kr, ^{99m}Tc, ¹¹¹In, ¹²³I, ¹²⁵I, ¹³¹I, ¹³³Xe, ¹⁵³Sm, and ²⁰¹Tl, or in general all detectable radioactive isotopes irrespective of their type of emitted radiation. The radiotracer molecules are delivered to the biological cells in the specimen and undergo binding or cell uptake. The specimen may comprise isolated cells or cells absorbed on a filter means, or a tissue section. Examples for cells are mammalian cell lines, primary cells, or induced pluripotent stem cells. Moreover, the cell culture or the cells comprised in the tissue section may exhibit cancerous characteristics. The radiotracer molecules are selected depending on the specimen or vice versa.

According to an embodiment, the specimen carrier and the receptacle are connectable via threads on at least one of the specimen carrier and the receptacle, and the device further comprises a sealing member for sealing the receptacle against the specimen carrier.

In an embodiment, at least one of the receptacle and the specimen carrier are made from a plastic material.

Examples for a suitable plastic materials polyamide (PA), polyethylene (PE), polyether ether ketone (PEEK), Poly (methyl methacrylate) (PMMA), polypropylene (PP), polytetrafluoroethylene (PTFE), polyurethane (PU), polyvinyl alcohol (PVA), polyvinyl chloride (PVC), and any other kind of plastic material that allows a substantial transmission of radiation, i.e. electromagnetic radiation such as visible light, ultraviolet, x-ray and gamma radiation and particle radiation, and does not absorb the radiotracer molecules for reducing background radiation in the collected image. The plastic material is formed, for example, by 3D printing, injection molding, or milling for fabricating at least one of the receptacle and the specimen carrier. Further, it is thinkable that the plastic material is compatible with one or more common sterilization methods, such as exposure to saturated hot steam, ethanol or isopropanol, gamma radiation, or ethylene oxide.

According to the present invention, a system is provided that comprises a camera configured to detect radiation and a device with the inventive features or additional features as defined above. Therein, the camera is arranged in proximity of the receptacle of the device such that radiation coming from the specimen can be detected by the camera.

Correspondingly, in its embodiments, the above-described system comprises additional features as described with regard to the device.

According to an embodiment, the camera comprises one or more detectors configured to collect the radiation coming from the specimen.

It is conceivable that the camera may be configured to detect various types of emission sources such as visible light emitted from fluorescence sources or radiation emitted from radioactive isotopes, i.e. gamma radiation or particle radiation. On that account, the one or more detectors of the camera are selected according to the emission source from which emitted radiation is detected, e.g. the radiotracer molecules which the cells in the specimen are saturated with.

According to another embodiment, the camera comprises one or more detectors and one or more collimators in front of each detector of the one or more detectors configured to collect the radiation coming from the specimen.

In a further embodiment, the camera comprises a ring-shaped detector, and the receptacle is positioned in the center of the detector to collect radiation coming from the specimen. It is conceivable that the device can be modified for any type of ring-shaped detector for detection of radiation coming from the specimen. It is conceivable that the camera further comprises means for 3D data reconstruction and means for data presentation to produce an image of the receptacle and the specimen on a screen of the camera.

It is conceivable that the radiation coming from the specimen, and more particularly, coming from the radiotracer molecules accumulated on the cells absorbed on filter paper, can be detected by the camera through its detectors.

The system enables the direct observation of the accumulation of the radiotracer molecules to the biological cells in the specimen by detecting the radiation coming from the cells. The measuring of the level of radioactivity in the cells can then lead to insights into the underlying accumulation mechanisms such as cell uptake and saturation. Hence, the system according to the present invention with the above-described features provides automation and high-throughput capabilities for efficient and reliable testing of drug-cell interaction.

Accordingly, in yet another embodiment, the camera is a nano or micro positron emission tomography, PET, camera, or a nano or micro single-photon emission computed tomography, SPECT, camera.

Hence, the system with above-defined features allows to directly observe the accumulation of the radiotracer molecules to the biological cells in high spatial and temporal imaging resolution. It is conceivable that nano or micro PET, nano or micro SPECT, or any other similar imaging modality allows dynamic or static imaging modalities.

According to an embodiment, the system further comprises a light source or an x-ray source, and another camera, wherein the light source or the x-ray source and the other camera are arranged in proximity of the receptacle such that light emitted from the specimen or x-ray radiation transmitted through the specimen can be detected by the other camera.

It is conceivable that the light source may emit ultraviolet or visible light.

According to an embodiment, the other camera comprises a detector that is configured to detect light from fluorescence emission or x-ray radiation.

It is thinkable that, in case the system comprises a light source for ultraviolet or visible light emission, the radiotracer molecules the cells in the specimen are saturated with may comprise a molecule for fluorescence, i.e. a fluorophore. It is conceivable that, in case the camera is configured to detect light from fluorescence emission, the camera may further comprise a filter set for fluorescence imaging, e.g. a FITC filter set. The fluorescently emitted light may be visible light and hence, the detector of the camera may be configured to detect visible light. Moreover, it is thinkable that the other camera may be ring-shaped and the receptacle is positioned in its center.

In another embodiment, the x-ray source and the other camera are components of an x-ray computed tomography, CT, camera.

Consequently, the system with the above-described features allows for combined functional and morphological imaging, which can be used, for example, to improve the imaging resolution and to produce 3D images of the specimen.

The present invention further provides a use of a device with the inventive features or additional features as defined above in nano or micro PET, or nano or micro SPECT, or any other camera suitable for detecting radiation coming from the specimen.

Accordingly, in its embodiments, the above-described use comprises additional features as described with regard to the device and the system.

Since the device described-above is used in nano or micro PET, or nano or micro SPECT, the accumulation of the radiotracer molecules to the biological cells in the specimen can be observed directly with high spatial and temporal imaging resolution. The measuring of the level of radioactivity in the cells can lead to insights into the underlying accumulation mechanisms. No additional procedures - such as complicated manual optical-investigation, washing or centrifugation/re-suspension steps- are needed before, during, or after data acquisition with a nano or micro PET, or nano or micro SPECT camera that would require intervention of experimenting personnel and that would put the cells under additional stress. Hence, the use according to the present invention allows for efficient and reliable testing of drug-cell interaction in an automatized and high-throughput approach.

According to the present invention, a method for testing accumulation of radiotracer molecules to biological cells with a system having the inventive features or additional features as defined above is provided. Therein, the method comprises the steps of loading the specimen carrier of the device of the system with a specimen, inserting the specimen carrier into the receptacle of the device through the opening of the receptacle; filling a fluid comprising the radiotracer molecules into the interior of the receptacle through the inlet of the receptacle so that the specimen is in connection with the fluid after loading of the specimen carrier with the specimen and inserting the specimen carrier into the receptacle. The method further comprises flushing the receptacle with a fluid not comprising the radiotracer molecules and detecting radiation coming from the specimen with the detectors of the camera of the system.

Consequently, in the method according to the invention, the accumulation of the radiotracer molecules to the biological cells in the specimen can be observed directly by detecting the radiation, i.e. particle radiation or emitted electromagnetic radiation, coming from the cells in the specimen, and more particularly, coming from emitting molecules the cells are saturated with. Electromagnetic radiation emitted from the specimen may be visible light, ultraviolet, x-ray or gamma radiation. In case the radiotracer molecules comprise radioactive radionuclides or isotopes, the measuring of the level of radioactivity in the cells can lead to insights into the underlying accumulation mechanisms. No additional measures - such as complicated manual optical-investigation, washing or centrifugation/re-suspension procedures - are needed before, during, or after detection that would require intervention of experimenting personnel and that would put the cells under additional stress. Hence, the method according to the present invention allows for efficient and reliable testing of drug-cell interaction in an automatized and high-throughput approach.

In an embodiment, the filling of the fluid into the interior of receptacle is performed by use of a pump means of the system connected with the inlet of the device. The method then further comprises directing the fluid through the receptacle and past the specimen by use of the pump means.

Hence, blood flow is mimicked, which allows for diffusive and convective delivery of the radiotracer molecules and nutrients to the specimen. Although the accumulation of radiotracer molecules to the cells present in the specimen is tested in-vitro, the method provides a more natural environment for the cells. This improves the informative value of the testing results. It is conceivable that the receptacle can be flushed with a fluid without any radiotracer molecules by use of the pump means, for example, before starting the testing or after a predetermined waiting time after filling the radiotracer molecules into the receptacle. Hence, the radiation, i.e. particle radiation or emitted electromagnetic radiation, coming from the cells can be detected in a state where the receptacle is filled with a fluid either with or without comprised radiotracer molecules, respectively.

In another embodiment, the detecting of radiation coming from the specimen with the camera is performed dynamically and starting before, during, or directly after the filling of the fluid comprising the radiotracer molecule into the interior of the receptacle.

Since the detecting of the radiation, i.e. particle radiation or emitted photons, is performed dynamically and started before, during, or directly after filling the radiotracer molecules into the receptacle, the cell saturation curve with the radiotracer molecules can be observed. No additional experiments are needed to assess equilibrium time and binding kinetics. It is conceivable to acquire the radiation data as a time sequence.

According to its embodiments the above-described method comprises additional features as described with regard to the device, system, and use.

In the following detailed description, preferred embodiments of the invention are described with reference to the enclosed drawing in which
- Fig. 1: shows a device for testing accumulation of radiotracer molecules to biological cells according to an embodiment of the invention in a side view;
- Fig. 2: shows the device for testing accumulation of radiotracer molecules to biological cells according to the embodiment of the invention in a front view;
- Fig. 3: shows a specimen carrier of the device for testing accumulation of radiotracer molecules to biological cells according to the embodiment of the invention in an exploded view;
- Fig.4A and Fig. 4B: show a device for testing the accumulation of radiotracer molecules on biological cells according to an embodiment of the invention;
- Fig. 5: shows a system for testing accumulation of radiotracer molecules to biological cells according to an embodiment of the invention;
- Fig. 6: shows an approach for loading cells on the filter paper of the device;
- Fig. 7: shows results of examples of accumulation tests of radiotracer molecules to biological cells according to an embodiment of the invention.

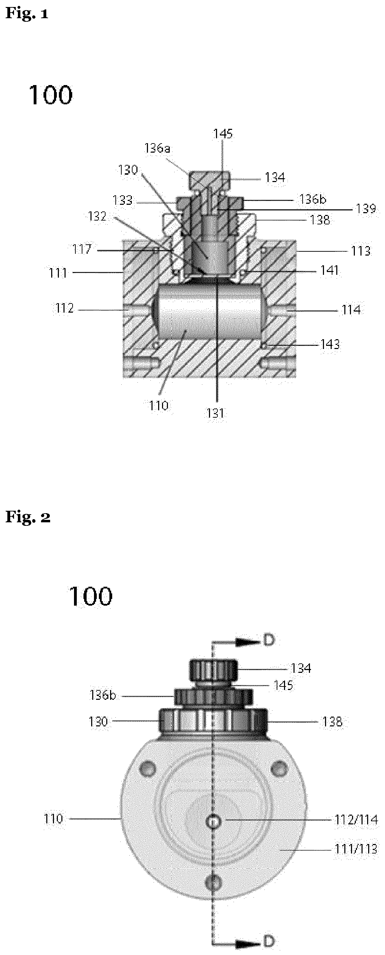

Figures 1 - 3 show a device 100 for testing the accumulation of radiotracer molecules on biological cells, i.e. for testing cells uptake and saturation, according to an embodiment of the invention. The device 100 comprises a receptacle 110 having an inlet 112, an outlet 114, and an opening 117 for receiving a specimen carrier 130. The device 100 further comprises the specimen carrier 130, wherein the specimen carrier 130 comprises an inner space that can accommodate the specimen, i.e. a filter paper 132 with absorbed cells, and is configured such that after loading of the specimen into the inner space of the specimen carrier 130 and insertion of the specimen carrier 130 into the receptacle 110, the specimen is in fluid connection with the interior of the receptacle 110 through the filter paper 132.

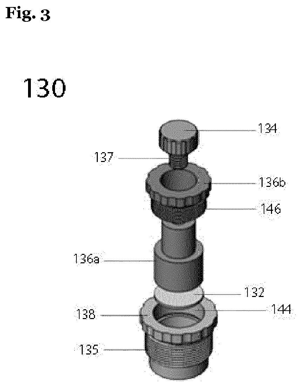

As shown in Fig. 1 and Fig. 3, the specimen carrier 130 comprises the shape of a tube with an opening at one end 131 and another opening at the other end 133 thereof, wherein the filter paper 132 is connected with the specimen carrier 130 so that the opening at the one end 131 is sealed. The opening at the other end 133 of the specimen carrier 130 comprises an inner thread 139 so that a lid 134 comprising a sealing member 145, here an O-ring, and an outer thread 137 can be screwed into the specimen carrier 130 for sealing the opening at the other end 133 of the specimen carrier 130. The one end 131 and the other end 133 of the specimen carrier 130 face each other so that the opening sealed with the filter paper 132 and the opening sealed with the lid 134 are opposite to each other and a line between the center of the filter paper 132 and the center of the lid 134 is a centerline of the specimen carrier 130.

Further, as shown in Fig. 1 and Fig. 3, the specimen carrier 130 comprises a first inset 136a, a second inset 136b, and a shell part 138. The first inset 136a has the shape of a tube with an opening at each of the two ends thereof. Therein, one part of the first inset 136a with one end has a first outer diameter and the other part of the first inset 136a with the other end has a second outer diameter, wherein the first outer diameter is larger than the second outer diameter to form a protrusion. Moreover, the opening at the other end of the first inset 136a comprises an inner thread 139 for screwing in the lid 134 so that the specimen can be held in the inner space of the first inset 136a. The second inset 136b has the shape of a tube with an opening at each of the two ends thereof. Since an inner diameter of the second inset 136b is equal to the second outer diameter of the first inset 136 the second inset 136b can hold the other part of the first inset 136a in its inner space and rest on the protrusion of the first inset 136a. On that account, the filter paper 132 has a diameter that is equal to or smaller than the first outer diameter of the first inset 136a and larger than the diameter of the opening at the one end of the first inset 136a. Furthermore, the shell part 138 comprises the shape of a tube, wherein one end of the shell part 138 has an opening with a protrusion and the other and of the shell part 138 has an inner thread 144 so that the second inset 136b having an outer thread 146 can be screwed into the shell part 138. The shell part 138 further has an inner diameter equal to the first outer diameter of the first inset 136a and an inner diameter equal to an outer diameter of the second inset 136b to hold the first 136a, the second 136b inset, and the filter paper 132 in its inner space. Therein, an inner diameter of the protrusion in the opening at the one end of the shell part 138 is smaller than the diameter of the filter paper 132 so that the filter paper 132 can rest inside and on the protrusion of the shell part 138. The filter paper 132 and the first inset 136a are inserted into the shell part 138 so that the filter paper 132 is stacked between the first inset 136a and the protrusion in the opening at the one end of the shell part 138. The second inset 136b is screwed into shell part 138 so that the second inset 136b presses against the protrusion on the first inset 136a and consequently, the first inset 136a presses the filter paper 132 against the protrusion in the opening at the one end of the shell part 138. Hence, the filter paper 132 is connected with the specimen carrier 130 so that the opening at the one end 131 of the specimen carrier 130 is sealed. Consequently, when the lid 134 is screwed into the into the first inset 136a and the second inset 1366 is screwed into the shell part 138 as described above, the inner space of the first inset 136a is closed by the filter paper 132 and the lid 134 for holding a specimen.

As shown in Fig. 1 - 3, the receptacle 110 of the device 100 comprises the shape of a tube with the inlet 112 at one end 111 and the outlet 114 at the other end 1113 thereof, wherein the opening 117 for receiving the specimen carrier 130 is positioned at a location on the shell of the tube-shaped receptacle 110. The shell of the tube-shaped receptacle 110 is flattened on the interior side of the receptacle 110 and from the one end 111 of the receptacle to the other end 113 at the location with the opening 117 for receiving the specimen carrier 130. The device 100 further comprises a sealing member 141 and the opening 117 of the receptacle 110 has a protrusion for holding the sealing member 141 so that upon screwing the specimen carrier 130 into the receptacle 110, the receptacle 110 is sealed against the specimen carrier 130.

The one end 111 and the other end 113 of the receptacle face each other so that the inlet 112 and the outlet 114 are opposite to each other and a line between the inlet 112 and the outlet 114 is a centerline of the receptacle 110. The one end 111 of the receptacle 110 comprises a plate with the inlet 112 and the other end 113 of the receptacle 110 comprises a plate with the outlet 114, wherein each plate is sealed against the shell of the receptacle 110 via a sealing member 143 and two or more connective members. Furthermore, the specimen carrier 130 and the receptacle 110 are connectable via an outer thread 135 on the specimen carrier 130 and inner thread (not shown) in the opening 117 of the receptacle 110. The specimen carrier 130 can be connected to the receptacle 110 in a way that the centerline of the receptacle 110 and the centerline of the specimen carrier 130 are orthogonal to each other.

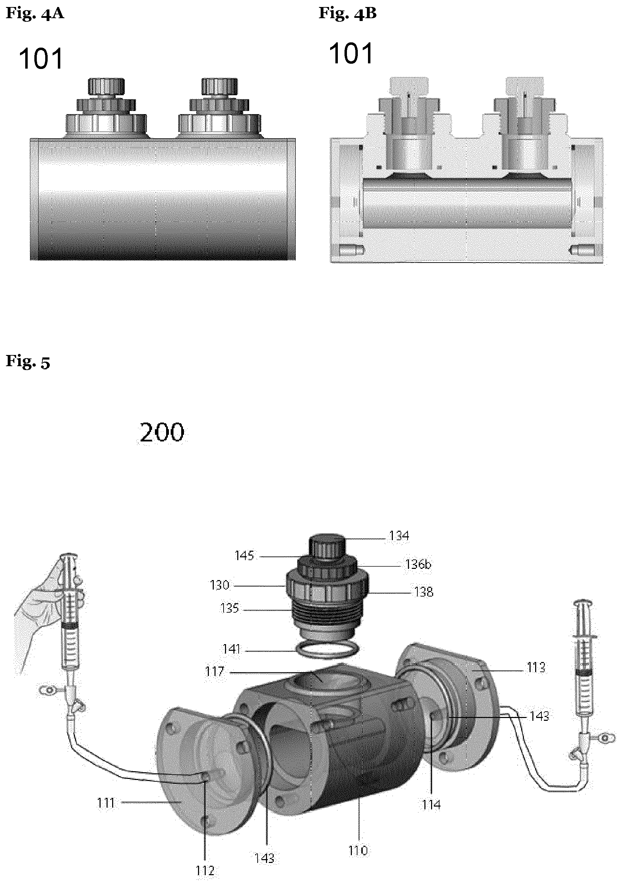

Fig. 4A and Fig. 4B show a device 101 for testing the accumulation of radiotracer molecules on biological cells, i.e. for testing cells uptake and saturation, according to an embodiment of the invention. This configuration of device 101 allows testing two different cells types for one radiotracer or two different studies, back to back, for one radiotracer. The device comprises a receptacle 110 and two openings 117 for receiving two specimen carriers 130, respectively.

Fig. 5 shows a system 200 according to an embodiment of the invention. The system 200 comprises the device 100 and a camera (not shown) configured to detect radiation, wherein the camera is arranged in proximity of the receptacle 110 of the device 100 such that radiation coming from the receptacle 110 can be detected by the camera. The system 200 further comprises a pump means 150, e.g. manually operated shielded syringes, connected with the inlet 112 of the device 100 to direct a fluid through the receptacle 110 and past a specimen in the specimen carrier 110. The inlet 112 of the receptacle 110 comprises the form of a hole and the pump means 150 comprises a shielded syringe and a tubing, wherein the tubing is plugged into the inlet 112. The pump means further comprises another shielded syringe and another tubing and the outlet 114 of the device 100 comprises the form of a hole, wherein the other tubing is plugged into the outlet 112. Consequently, the fluid can be injected into the receptacle 110 by use of the syringe connected to the inlet 112, directed through the receptacle 110 and past the specimen in the specimen carrier 130, and then taken up by the syringe connected to the outlet 114. Using the shielded syringes, a streaming of a fluid past the specimen can be simulated.

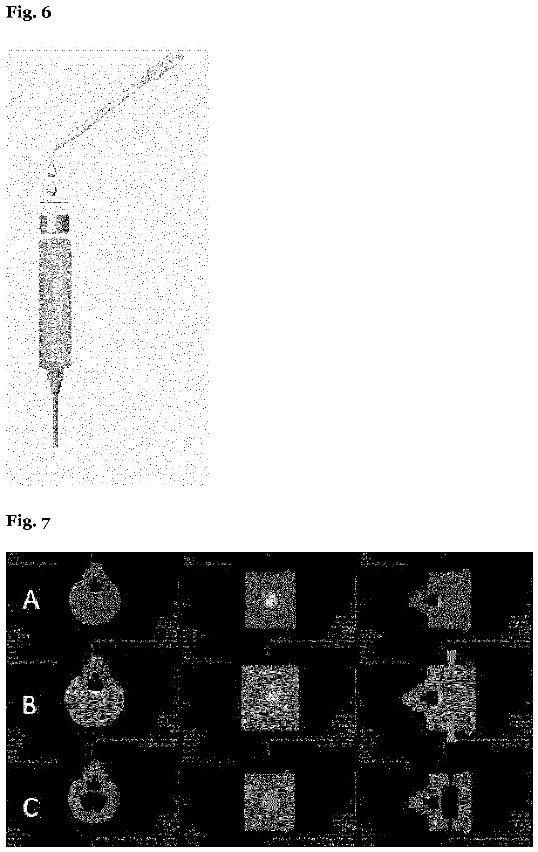

Fig. 6 shows an approach for loading cells on the filter paper 132 of the device 100. In case the specimen comprises cells absorbed on the filter paper 132, the system 200 is suitable for two ways of loading the cells on the filter paper 132. The first approach comprises externally absorbing the cells on the filter paper 132 and accommodating the filter paper with the absorbed cells 132 the specimen carrier 130. As shown in Fig. 6, a syringe body, e.g. a 10 cc syringe body, and HPLC solvent line filter head can be used for the first approach to absorb the cells on the filter paper 132. The second approach comprises adding the cells directly through the lid 134 of the specimen carrier 130, closing the inlet 112 of the device 100 and applying a slow vacuum pulse through the outlet 114 for a short duration in time, e.g. seconds, to remove any access of cell media.

The approaches disclosed herein generally serve for providing automation and high-throughput capabilities for efficient and reliable testing of drug-cell interaction.

According to an embodiment of the invention the device 100 is used in nano or micro PET, or nano or micro SPECT, or any other camera suitable for detecting radiation coming from the specimen.

Further, according to an embodiment of the invention, a method for testing the accumulation of radiotracer molecules on biological cells with the above-described system 200 is provided. The method comprises the steps of loading the specimen carrier 130 of the device 100 of the system 200 with a specimen and inserting the specimen carrier 130 into the receptacle 110 of the device 100 through the opening 117 of the receptacle 110. Subsequently, a fluid comprising the radiotracer molecules is filled into the interior of the receptacle 110 through the inlet 112 of the receptacle 110 so that the specimen gets in contact with the fluid after loading of the specimen carrier 130 with the specimen and insertion of the specimen carrier 110 into the receptacle 110. Radiation coming from the receptacle 110 is then detected with the camera of the system 200. More particularly, the camera may comprise one or more detectors to collect the radiation. The fluid is filled into the interior of receptacle 110 by use of pump means 150 of the system 200, e.g. manually operated shielded syringes, connected with the inlet 112 and the outlet 114 of the device 100, respectively. The method further comprises directing the fluid through the receptacle 110 and past the specimen by use of the pump means 150.

Detailed advantages of device 100, system 200, use, and method of the invention in comparison to conventional devices, systems, uses, and methods for cell studies are summarized in Table 1.

**Table 1**

| **Uptake study** | **Conventional method** | **Present invention** |
|---|---|---|
| **Number of test tubes** | 21 tubes per study | Small filter paper |
| **Count of cells** | Minimum 2 million | 100 - 500 K enough |
| **Radiotracer Concentration per study** | At least 7 different Concentration | One fixed Concentration |
| **Preparation time** | 3-4 hrs | 20 min |
| **Incubation time** | 1 hour | 20 min |
| **Centrifuging and Count time** | 4-6 hrs | 0 min |
| **Centrifuging** | For each tube | No need |
| **Dose Calibrator Counting** | Count each tube | Single image produce all needed information. |

In the following, examples of the invention are described with respect to a device, system, use, and method for testing accumulation of radiotracer molecules to biological cells. The examples disclosed herein generally shows how a device, system, use, and method according to the present invention provides automation and high-throughput capabilities for efficient and reliable testing of drug-cell interaction.

In the given examples, an above described system 200 with device 100 is provided. The filter paper 132 of the device 100 has a diameter of 16 mm, the receptacle 110 of the device 100 has a volume of 12.3 ml, and the specimen carrier 130 of the device 100 has a volume of 1.4 ml. The receptacle 110, the specimen carrier 130, and the filter paper 132 are fabricated from PMMA. The filter paper material-is a WHATMAN filter paper, i.e. a glass fiber filter paper produced from 100% borosilicate glass without the use of any adhesive or binder.

The system 200 further comprises a Nano-PET/CT camera (Mediso company, Budapest, Hungary), wherein the Nano-PET/CT camera is arranged in proximity of the receptacle 110 of the device 100 such that radiation or emission source coming from the receptacle 110 can be detected by the Nano-PET/CT camera The PET specifications of the camera are: gantry opening 16 cm; multiple animal imaging up to 4 x 60 g mice 2 x 500 g rats; sensitivity 8%; transaxial FOV 12 cm; detector crystal LSO 1.12 x 1.12 x 13 mm³; Noise Equivalent Count Rate for mouse NEMA 850 kcps @ 60 MBq; axial FOV 10 cm; spatial resolution with Tera-Tomo 3D OSEM 0.7 mm; Noise Equivalent Count Rate for rat NEMA 250 kcps @ 60 MBq; animal models mouse, rat, marmoset, guinea pig, rabbit; spatial resolution with FBP NEMA 1.25 mm. The CT specifications of the camera are: X-ray power up to 80 W; low-dose protocol down to 1 mSv for whole-body mouse; transaxial FOV 12 cm; axial FOV 12 cm; spatial resolution 30 µm at 10 µm voxel size; image reconstruction modified Feldkamp-type for real-time reconstruction, iterative for low-dose and low-noise applications.

The system 200 with the device 100 and Nano-PET/CT camera allows simultaneous detection of the level of radioactivity of the biological cells within the specimen and morphological cell imaging. However, it is conceivable that the device 100 can be used in any other nano-imaging modality having ring-shaped detectors that are suitable for collecting radiation coming from the specimen.

According to the examples, ¹⁸F-Fluorodeoxyglucose (¹⁸F-FDG), a glucose analogue with a ¹⁸F radionuclide, is used as radiotracer molecule. ¹⁸F-FDG is phosphorylated by hexokinase and taken up by cells that consume glucose. Metabolic uptake of the radioactive glucose molecules enables imaging regions of high glucose consumption in a specimen with a PET camera. The concentration of ¹⁸F-FDG imaged indicates the metabolic activity of the tissue as it corresponds to regional glucose uptake. Hence, ¹⁸F-FDG is particularly suitable to image cancerous cells in a specimen that generally show high metabolic activity.

In the two given examples, the cancerous cell cultures MCF-7 and MDA-MB-231 (Breast cancer), respectively, are used as specimens and are loaded onto the filter paper 132. The number of cells for each tested example was approximately 500,000. The filter paper 132 with the cells of the corresponding cell culture is placed into the specimen carrier 130 and the device 100 and system 200 is assembled and connected as described above. After assembly and connection, the device 100 is filled with saline (NaCl 0.9%) and a fluid with the ¹⁸F-FDG radiotracer molecules is injected. After injection, the solution is allowed to move slowly within the receptacle 110 for 20 min, allowing the radiotracer molecules to be delivered to the cells by convective/diffusive movement in analogue to physical movement, in particular, simulating blood vessel contractions. The radiation coming from the receptacle 110 either loaded with the MCF-7 or MDA-MB-231 cells is detected with the Nano-PET/CT camera of the system 200. The detection can be performed either statically or dynamically to observe the delivery of the radiotracer molecules to the cells and the cell uptake.

According to the examples, the fluid with ¹⁸F-FDG in the receptacle 110 is removed by flushing the receptacle 110 with saline after 20 min of running ¹⁸F-FDG inside the device 100 and a static image is taken with the Nano-PET/CT camera.

Fig. 7A and Fig. 7B present the results of the examples measured with the MDA-MB-231 and MCF-7 cells, respectively, and Fig. 7C presents the results of a control. The results of the examples show that the cell saturation or uptake and thus, the binding strength of the radiotracer molecules to the cells can be detected with the above-described system 200 according to an embodiment of the invention. As a control, the same process as described above is performed but without loading any radiotracers. The result of the control ensures that the radioactive radiotracer molecules have no real effect on the filter paper 132.

### List of reference signs

- 100: device
- 101: device
- 110: receptacle
- 111: one end of the receptacle
- 112: inlet
- 113: other end of the receptacle
- 114: outlet
- 117: opening
- 130: specimen carrier
- 131: one end of the specimen carrier
- 132: filter paper
- 133: other end of the specimen carrier
- 134: lid
- 135: outer thread of the specimen carrier
- 136a: first inset
- 136b: second inset
- 137: thread of the lid
- 138: shell part
- 139: inner thread of the specimen carrier
- 141, 143, 145: sealing member
- 144: inner thread of the shell part
- 146: outer thread of the second inset
- 150: pump means
- 200: system

## Claims

1. A device for testing the accumulation of radiotracer molecules on biological cells, comprising:
- a receptacle (110) having an inlet (112), an outlet (114), and an opening (117) for receiving a specimen carrier (130); and
- the specimen carrier (130) comprising means for holding a specimen and being configured such that after loading of the specimen carrier (130) with a specimen and insertion of the specimen carrier (130) into the receptacle (110), the specimen being in fluid connection with the interior of the receptacle (110), wherein the specimen carrier (130) or the specimen comprises a filter means (132).

2. The device of claim 1, further comprising a pump means (150) configured for a connection with the inlet (112) to direct a fluid through the receptacle (110) and past the specimen.

3. The device of claim 1 or 2, wherein the specimen carrier (130) comprises an inner space that can be closed by the filter means (132), and is configured such that after loading of the specimen into the inner space of the specimen carrier (130) and insertion of the specimen carrier (130) into the receptacle (110), the specimen is in fluid connection with the interior of the receptacle (110) through the filter means (132).

4. The device of anyone of claims 1 to 3, wherein the specimen carrier (130) comprises the shape of a tube with an opening at at least one end (131) thereof, wherein a filter means (132) can be connected with the specimen carrier (130) so that the opening at the at least one end (131) is sealed.

5. The device of anyone of claims 3 or 4, wherein the filter means (132) has a pore size such that the filter means (132) is substantially permeable for the radiotracer molecules and substantially non-permeable for the biological cells, wherein the radiotracer molecules comprise a chemical compound or a biomolecule with a radionuclide, and the specimen comprises the biological cells.

6. The device of anyone of claims 1 to 5, wherein the specimen carrier (130) and the receptacle (110) are connectable via threads (135) on at least one of the specimen carrier (130) and the receptacle (110), and the device further comprises a sealing member (141) for sealing the receptacle (110) against the specimen carrier (130).

7. The device of anyone of claims 1 to 6, wherein at least one of the receptacle (110) and the specimen carrier (130) are made from a plastic material.

8. A system comprising a camera configured to detect radiation and a device according to one of claims 1 to 7, the camera being arranged in proximity of the receptacle (110) of the device such that radiation coming from the specimen can be detected by the camera.

9. The system of claim 8, wherein the camera is a nano or micro positron emission tomography, PET, camera, or a nano or micro single-photon emission computed tomography, SPECT, camera, or any other camera suitable for detecting radiation coming from the specimen.

10. The system of claim 8 or 9, wherein the system further comprises a light source or an x-ray source, and another camera, wherein the light source or the x-ray source and the other camera are arranged in proximity of the receptacle (110) such that light emitted from the specimen or x-ray radiation transmitted through the specimen (110) can be detected by the other camera.

11. The system of claim 10, wherein the x-ray source and the other camera are components of an x-ray computed tomography, CT, camera.

12. Use of a device according to one of claims 1 to 7 in nano or micro PET, or nano or micro SPECT.

13. A method for testing accumulation of radiotracer molecules to biological cells with a system according to one of claims 8 to 11, the method comprising:
loading the specimen carrier (130) of the device of the system with a specimen;
inserting the specimen carrier (130) into the receptacle (110) of the device through the opening (117) of the receptacle (110);
filling a fluid comprising the radiotracer molecules into the interior of the receptacle (110) through the inlet (112) of the receptacle (110) so that the specimen is in connection with the fluid after loading of the specimen carrier (130) with the specimen and inserting of the specimen carrier (110) into the receptacle (110); and
detecting radiation coming from the specimen with the camera of the system.

14. The method of claim 13, wherein the filling of the fluid into the interior of the receptacle (110) is performed by use of a pump means (150) of the system connected with the inlet (112) of the device, and the method further comprises directing the fluid through the receptacle (110) and past the specimen by use of the pump means (150).

15. The method of claim 13 or 14, wherein the detecting of radiation coming from the specimen with the camera is performed dynamically and starting before, during, or directly after the filling of the fluid comprising the radiotracer molecule into the interior of the receptacle (110).
